# EUROPEAN PATENT SPECIFICATION

(11) **EP 0 519 958 B1**
(45) Date of publication and mention of the grant of the patent: **19.10.1994**
(21) Application number: 91905582.2
(22) Date of filing: 08.03.1991
(51) Int. Cl.: A61N 5/04, A61N 5/02

(54) **Apparatus for the surgical treatment of prostate tissue by thermal effect, using a urethral microwave probe**
Vorrichtung zur chirurgischen Behandlung der Prostata mittels thermischer Wirkung unter Verwendung einer Mikrowellen emittierenden Urethralsonde
Appareil de traitement chirurgical de tissu prostatique, par effet thermique, utilisant une sonde urétrale à émission de micro-ondes

(30) Priority: 12.03.1990 FR 9003121
(43) Date of publication of application: 30.12.1992
(73) Proprietor: TECHNOMED MEDICAL SYSTEMS, 69120 Vaulx-en-Velin (FR)
(72) Inventor: CATHAUD, Muriel, F-69780 Vénissieux (FR); LACOSTE, François, F-69006 Lyon (FR); DEVONEC, Marian, F-01700 Miribel (FR)
(74) Representative: Portal, Gérard
(86) International application number: EP9100444
(87) International publication number: WO9113650

(56) References cited:
- EP-A- 0 246 176
- EP-A- 0 248 758
- EP-A- 0 370 890
- WO-A-89/02292
- WO-A-89/05609
- US-A- 4 311 154
- US-A- 4 612 940
- Rick McGuire:"Transurethral hyperthermia probe for BPH: Trial's goal is to top 80% success", in Medical Tribune, Thursday, March 31, 1988, pages 3,13,14
- T.Harada et al.:"Microwave surgical treatment of diseases of prostate", Urology, Dec.85, Vol.26,No.6,pp.572-576
- PROCEEDINGS OF THE IEEE, vol. 72, no. 2, February 1984, New York, US; pages 224-225; Hao LING et al.: "Frequency optimization of focused microwave hyperthermia applicators"

## Description

### FIELD OF THE INVENTION

The present invention relates to a method and apparatus for the surgical treatment of tissues by thermal effect, and in particular, the prostate, using a urethral microwave-emitting probe means.

### BACKGROUND OF THE INVENTION

European patent 0,248,758 describes a microwave applicator which is inserted in the rectum for treating the prostate by hyperthermia. The microwave applicator is kept in position inside the rectum by a laterally projecting balloon, meaning that special protection of the rectum is required. Preferably, for heating the prostate, means are provided for reflecting the emitted microwaves which are then concentrated at the side of the body opening opposite to the balloon. Ducts are provided in the microwave emitting rectal applicator for circulating a cooling liquid (see page 5, lines 1 to 5).

The cooling liquid protects the rectal walls in contact with the microwave antenna from damage. Moreover, the presence of the reflecting means is absolutely necessary to prevent the antenna from emitting in all directions, when the radiation is only required to be directed onto the prostate tissues.

European patent 0,246,176 describes a catheter having an antenna equipped with a temperature sensor which is inserted into the urethra through to the bladder, for controlling the temperature reached in the urethra through use of the microwave applicator described in the above-cited European patent 0,248,558.

From a practical point of view, the design of the microwave applicator described in European patent 0,248,758 for introduction in the rectum, is not satisfactory in that the application lacks accuracy since the prostate is remotely-irradiated.

It is furthermore essential with this method to prevent the emission of microwaves in all directions, this implying an emitting antenna of very special design equipped with reflecting means for directing the radiated microwaves in only one direction, namely towards the prostate.

Also, and unquestionably, with such a device, it is quite difficult, if not impossible, to protect the sphincter, and more generally to protect the surrounding tissues. This being especially so in the case of bladder treatment.

Finally, no working frequency is disclosed.

European patent 0,105,677 describes such a microwave antenna device for hyperthermia therapy designed to be introduced in the tract or the lumen, in which device the antenna assembly is placed inside a thin supple polymer film, without any means of protecting the sensitive tissues from the heat. The working frequency is broadly disclosed as ranging between 300 and 3,000 MHz, for instance 915 MHz (page 4, lines 7 and 24).

Patent document WO-A-81/03616 to Bicher describes yet again a microwave antenna system for hyperthermia therapy, which is inserted into a body cavity and used for the treatment of cancer and similar. Typical examples of inserting apertures are the mouth and upper throat.

The introductory part of said patent document cites the document J. Microwave Power 14(2) : 167-171 (1979) relative to an applicator working at a frequency of 400 or 915 MHz, introduced through the rectum for irradiation of the prostate. It is specified that said irradiation treatment involves a burning of the tissues which are close to the electrode.

According to said document, the characteristic of this irradiation treatment is that it is carried out at a lower frequency and at a higher wavelength to improve penetration into the tissues to depths of 5 to 6 cm. Such deep penetration permits extensive irradiation of large tumors in the bowels or similar.

The revue "Proceedings of the 13th Annual Northeast Bioengineering Conference, Philadelphia, March 12-13, 1987, pages 390-393, Ryan" describes the pretreating by microwave-induced hyperthermia of patients suffering from biliary obstruction due to cancer, by inserting a catheter into the choledoch to allow drainage in an invasive way. The catheter works at 915 MHz.

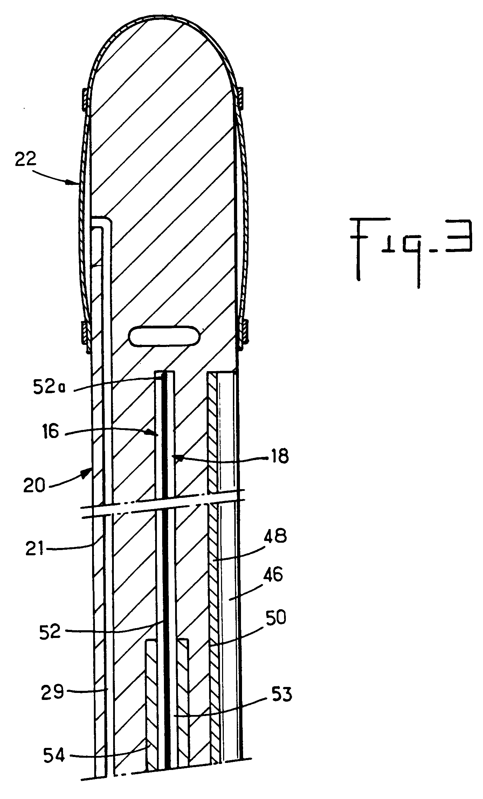

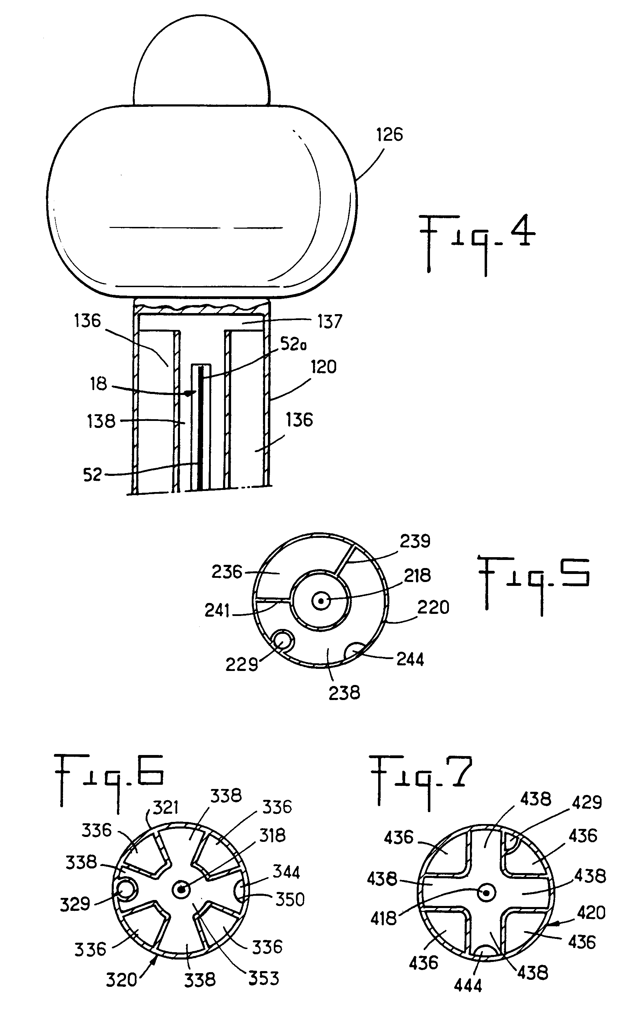

Tests have been carried out with the same antenna in various media, as reported in Figure 5. In the case of tests B and D, the medium was a saline solution, and it is underlined in the "Discussion" part that when the medium is changed from air to a conductive medium such as a saline or bile medium, the performances decrease dramatically, which rather discourages the specialist from using a saline solution in microwave-induced hyperthermia.

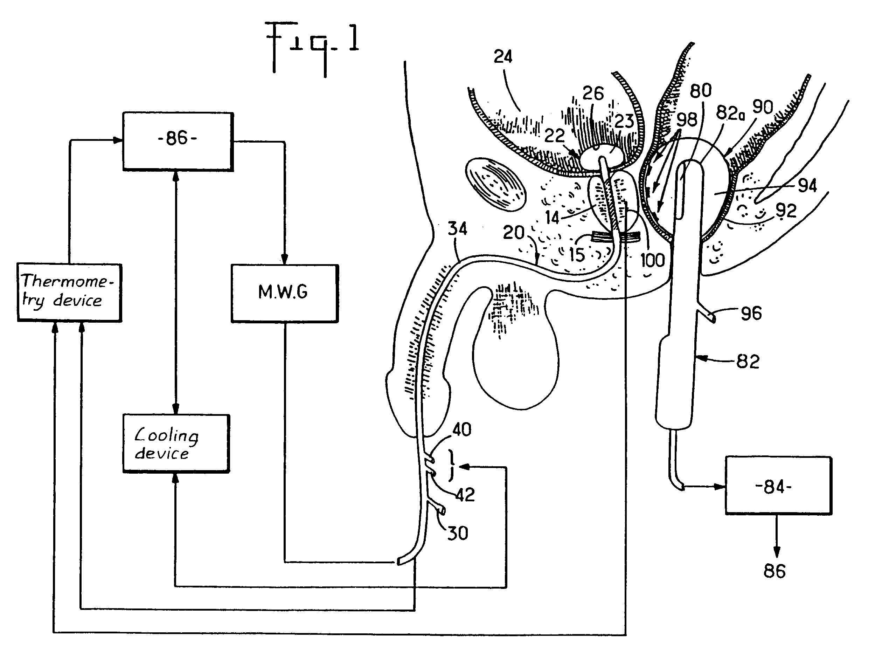

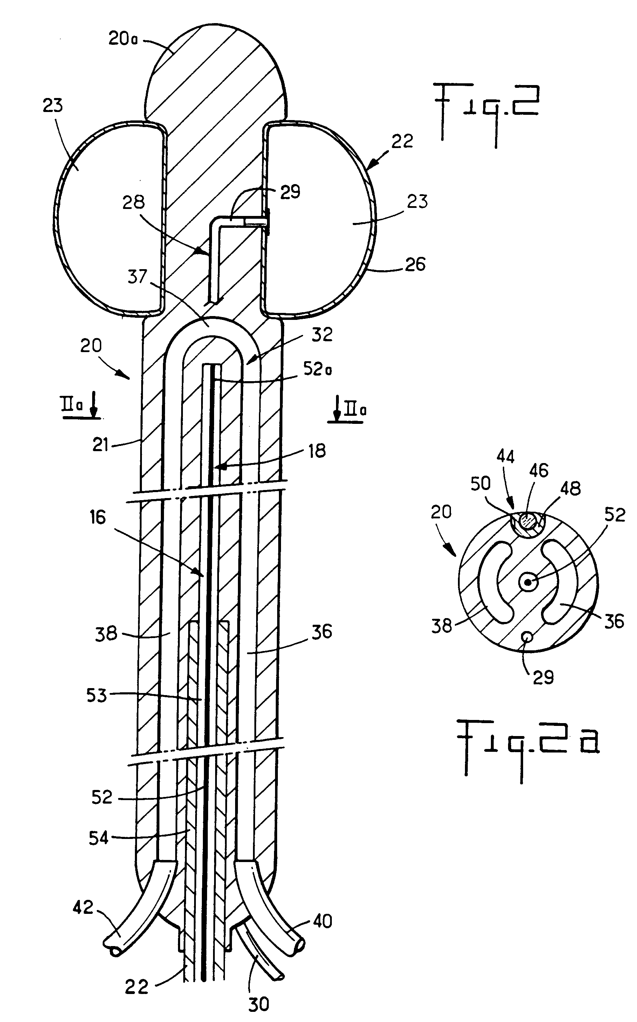

Also, it is known from Proceedings of the IEEE, 72 (1984), February, No. 2, 224-225, the advantage of using focused microwave hyperthermia applicators. It is also disclosed obtention of an optimal frequency for a fixed aperture size and a given penetration depth. Figure 2 discloses optimal frequencies for given penetration depth of 2, 4 and 6 cm at 3,000 ; 1,500 and 915 MHz.

SCHEIBLICH in the Journal of Microwave Power, 17(3), 1982, pages 203-209, notably page 204, is emphasizing the advantage of choosing a frequency at 433.9 MHz of the microwave antenna for providing a better penetration depth of the induction field and for requiring a maximum power reduced at about 35-55 watts for a successfull hyperthermia of the prostate.

Medical Tribune's article of March 31, 1988 is disclosing a working frequency of 915 MHz like STERZER US-A-4,311,154. The frequency of 915 MHz is also cited in EP-A-370,890 (TECHNOMED).

Furthermore, TECHNOMED's intermediate application EP-A-370,890 is disclosing an urethral probe provided with cooling means.

### SUMMARY OF THE INVENTION

It is now a main object of the present invention to solve the new technical problem consisting in providing a solution permitting a more accurate surgical treatment of tissues by thermal effect, in particular of the prostate.

It is a further main object of the present invention to solve the above new technical problem, particularly in the thermal effect treatment of the prostate, without the need to use special probe means emitting in only one direction.

It is a further main object of the present invention to solve the new technical problem, consisting in providing a solution permitting to precisely generate the microwaves in a relatively narrow penetration field, thereby avoiding to damage the tissues surrounding the tissues to be treated by thermal effect, these surrounding tissues necessitating not to be reached by the microwaves used to perform the thermal effect treatment.

It is another main object of the present invention to solve the new technical problem consisting in providing a solution permitting to perform the treatment of tissues by thermal effect in a localized manner, while providing a possibility of adapting the thermal effect treatment to the shape and the size of the tissues to be treated.

It is another object of the present invention to solve the aforesaid new technical problems in a semi-invasive way, namely by using the natural body cavities or body lumens.

All these technical problems are solved for the first time by the present invention, extremely simply, inexpensively, with greater accuracy and reliability in a manner exploitable on an industrial scale.

According to the present invention, the thermal effect is preferably a "thermotherapy", that means that the tissues are treated by heat energy at a temperature higher than 45°C and preferably ranging between about 45°C and not more than about 85°C, during a period of time sufficient to reach necrosis of the tissues submitted to the thermal effect.

Thermal effect treatment according to the preferred invention embodiment is fully different from the hyperthermia treatment of the prior art, since hyperthermia is limited to a temperature range lower than 45°C and generally of 40°C - 43°C. The invention is based on the discovery of unexpected positive therapeutical effects provided by a heating of tissues at a temperature higher than 45°C versus a heating of said tissues at a temperature lower than 45°C. But, the invention can also be used to perform a hyperthermia treatment.

Accordingly, the present invention provides an apparatus for the surgical treatment of prostate tissues by thermal effect, comprising an urethral emitting probe adapted to be inserted in the urethra, having a front end and a rear end, said emitting probe comprising the microwave antenna means located intermediate or in the vicinity of said front end, said microwave antenna means being connected to an external microwave generating device, said urethral emitting probe comprising cooling means fed with a cooling medium, and being capable of preventing burns on the walls of the prostatic urethra in contact with the urethral probe means, said external microwave generating device comprises means for generating microwaves at a frequency of 1300 MHz ± 50 MHz, and a power effective to heat permanently said tissues to a predetermined temperature for a period of time sufficient to treat said tissues by thermal effect.

According to a specific feature, the cooling means are extending up to the full length of the microwave antenna means.

According to another feature, an inflatable balloon means is located between said microwave antenna means and said front end, and means for inflating said balloon means, said balloon means reaching the bladder when said urethral probe is positioned in the urethra.

According to a preferred embodiment, said means for inflating said balloon means comprise an electroconductive liquid forming a radio reflecting screen capable of reflecting the microwaves emitted by said microwave antenna means, thereby protecting the bladder from being damaged by said microwaves.

According to another specific embodiment, said power is effective to treat said tissues by heat energy at a temperature higher than 45°C and preferably ranging about 45°C and not more than 85°C, during a period of time sufficient to reach a necrosis of the tissues submitted to the thermal effect to perform thermotherapy.

According to another specific embodiment, said power is effective to treat said tissues by heat energy at a temperature range lower than 45°C and generally of 40°C-43°C to perform a hyperthermia treatment.

Advantageously, the urethral inflating means comprise a liquid medium.

According to another embodiment, said apparatus comprises:
a) a urethral emitting probe having a front end and a rear end, capable of being inserted in the urethra, said urethral emitting probe comprising a microwave antenna means located intermediate or in the vicinity of said front end, said microwave antenna means being connected to an external microwave generating device ;
b) a rectal probe means comprising control means for controlling the positioning of the urethral probe means;
c) a central control device receiving the information provided by the control means for controlling the positioning of the urethral probe means,
d) correcting means for correcting the positioning of the urethral probe means, and
e) said external microwave generating device comprising microwaves generating means adapted for generating microwaves at said frequency and at a power effective to heat said tissues to a predetermined temperature for a period of time sufficient to treat said tissues by thermal effect.

According to another embodiment, said rectal probe comprises an echographic probe connected to a image forming means to facilitate the positioning of the urethral probe means, thereby having an echographic control of the surgical treatment of said tissues.

According to another specific feature, said rectal probe comprises liquid medium feeding means for feeding a liquid medium, capable of providing heat protection of the rectal walls.

Advantageously, said rectal probe comprises an echographic probe connected to a screen equipped echograph and to a central control device for receiving the information provided by said echographic probe and controlling the positioning of the urethral probe means,
- correcting means for correcting the positioning of the urethral probe, means,
- said external microwave generating device comprising microwaves generating means adapted for generating microwaves at a frequency and at a power effective to heat said tissues to a predeterminined temperature for a period of time sufficient to treat said tissues by thermal effect, thereby having an echographic control of the surgical treatment of said tissues.

According to another embodiment, said urethral probe comprises at least one urethral temperature sensor located in the vicinity of the microwave antenna means for detecting urethral temperature,
- said central control device comprises power and cooling temperature adjusting means to adjust one or both of the microwaves power and of the cooling temperature of the cooling means, when the temperature detected selected from the urethral temperature and from the rectal temperature is not conform to said predetermined temperature range.

According to another embodiment, the invention provides an apparatus wherein the rectal probe is provided with a rectal inflatable balloon element mounted around the rectal probe on its front end and with rectal inflating means comprising an inflating liquid medium and feeding means for feeding said liquid medium through said rectal probe inflating means, thereby providing heat protection of the rectal walls. This inflating liquid can be advantageously a radioreflecting liquid in particular by being electroconductive.

According to a further embodiment, the invention provides an apparatus wherein the rectal probe is provided with radio reflecting means to protect the rectal walls from heat.

According to another embodiment, the present invention provides an apparatus, wherein said urethral temperature sensor comprises a plurality of strands of optical fibers placed in radially and/or longitudinally offset fashion in the longitudinal direction of the urethral emitting probe.

According to a further embodiment, the present invention provides an apparatus, wherein said urethral probe means comprises cooling means fed with a cooling medium, said cooling means extending up to the full length of the microwave antenna means, said cooling means comprising cooling ducts arranged symmetrically with respect to a symmetry plane of said urethral emitting probe, said urethral temperature sensors and said balloon inflating means being located opposite in `he same symmetry plane passing through the axis of the urethral probe means.

According to the present invention, it is possible to treat benign or malignant tissues by coagulation of cytoplasmic proteins or by a myorelaxing effect on the muscular fibers, or a bacteriostatic effect, or to treat localized cancers of the bladder or prostate, or prostatitis or cystitis, and more generally to perform the treatment of benign prostatic hypertrophy.

Further, the ability to cool the urethra allows for more effective heat delivery to the centre of the prostatic lateral lobes with preservation of the periurethral tissues.

This ensures an increase therapeutic tissue destruction within the prostate leading to a more effective improvement in voiding status that has been found with the prior art prostatic microwave hyperthermia devices.

Furthermore, according to the invention, process and apparatus, the overall operations are accomplished in most patients with a single session, anesthesia free treatment.

Further, with the help of a balloon self-retaining device provided at the front end of the urethral probe, it is rendered possible a quick and precise positionning of the microwave antenna means within the prostatic urethra in relation to the bladder neck.

Furthermore, with the help of the cooling means for cooling the urethral probe, it is possible to lower the temperature of the urethra walls during treatment with accuracy by controlling the flow of the cooling medium and/or the temperature of the cooling medium.

Further, the monitoring of the temperature of the urethra wall is safely and accurately effected with the presence of temperature sensors combined with the uretral probe. Additionally by incorporating also temperature sensors with the rectal probe, it is possible to monitor the temperature at the rectal wall. By providing a central control module, notably comprising a technical computer, it is possible for the physician to control precisely the power output of the unit in response to the temperature measured by the urethral and/or rectal thermosensors. The achieved urethral and/or rectal temperatures and power output are displayed in real time and recorded by the computer for subsequent analysis. Print out of the treatment parameters and temperature tracings are preferably available for inclusion in each individual patient record.

It is also preferred to have safety devices incorporated in the invention apparatus, to prevent the excessive delivery of energy. These safety devices preferably include a thermometry feed-back system which maintains the temperature at not more than 44.5°C at the level of the urethral walls as measured by the urethral regulating thermosensors, and at not more than 42.5°C, at the level of the rectal walls as measured by the rectal thermosensors.

It is preferred that the thermometry system is of the fiber optical type designed to accurately measure the temperature within the microwave field.

According to a specific feature, the rectal probe is provided with several optical thermosensors, in particular at least three optical thermosensors disposed in radially and/or longitudinally offset fashion in the longitudinal direction of the probe means, in such a way as to detect the temperature in various radial and/or longitudinal directions of the probe means.

According to an advantageous variant of embodiment, said apparatus comprises heat protecting means for protecting from the heat the sensitive tissues other than the tissues to be treated, said means preferably comprising means forming radioreflecting screen. Said apparatus may also be characterized in that one of the means forming radioreflecting screen is placed at the level of an organ to be protected, such as the bladder, the sphincter.

Preferably, another part of the means forming radioreflecting screen protects the rectal wall from high temperatures.

According to a particularly advantageous embodiment of the invention, the means which, according to the invention, form radioreflecting screen, contain a radioreflecting liquid medium. Advantageously, said radioreflecting liquid medium is an electrically conductive liquid reflecting the microwaves.

According to a variant embodiment, the radioreflecting screen-forming means according to the invention comprise at least one wire, parallel to the mean electrical field.

Preferably, one part of the means forming radioreflecting screen according to the invention is at the level of the prostate to be treated, such as the bladder, the sphincter.

According to another particularly advantageous embodiment of the invention, said means forming radioreflecting screen and protecting the bladder may comprise a urethral balloon provided on the front end of the antenna, urethral inflating means being provided for inflating said balloon with a radioreflecting conductive solution.

According to a variant embodiment, the screen-forming means protecting the bladder comprise a conductive solution injected directly into the bladder, said solution being preferably hypertonic.

According to another particularly advantageous embodiment of the invention, another part of said screen-forming means is fast with a rectal probe means, for protecting the rectal wall from high temperatures.

Thus, according to a variant embodiment of said means forming protection screen for the rectal wall, said means can comprise a radioreflecting liquid medium, in particular one introduced into a rectal balloon provided around the front end of the rectal probe means.

Means for immobilizing the rectal probe means in position can be provided, said means preferably permitting a rotation of said probe means around its axis. Said immobilizing means preferably comprise a rectal balloon, for example in latex, which surrounds the rectal probe means, and inside which said probe means can be readily oriented from the outside.

According to one particular variant of embodiment, said microwave generating device comprises an inner conductor comprising a metallic cable coaxial to the probe means covered by an electrically insulating sleeve over its whole length, and an outer conductor extending over part of the length of the inner conductor, the emitting microwave antenna means being defined by the front part of the inner conductor, which ends at an intermediate level of the emitting probe means.

According to yet another particularly advantageous variant of embodiment, said temperature sensing means comprise a fiber-optic thermometer, heat-insulated from the cooling circuit, preferably comprising several strands of fibers disposed in radially and/or longitudinally offset fashion in the longitudinal direction of the probe means, in such a way as to detect the temperature in various radial and/or longitudinal directions of the probe means.

It is therefore obvious that the apparatus according to the invention, the emitting probe means can be positioned with great accuracy since it is inserted directly into the prostate along the natural urethral passage, thus preventing the sphincter from being damaged.

Furthermore, by setting the frequency of the microwave means at a value of about 1,300 MHz ± 50 MHz, it is unexpectedly obtained a very efficient thermal effect treatment of the prostate in the centre thereof, enabling in a single session to treat the full volume of the prostate by thermal effect.

Also, owing to the radioreflecting screen-forming means according to the invention, it becomes possible to protect efficiently the bladder as well as the rectal walls. It is possible, according to the invention, to use a probe means which preferably emits in one given direction.

Understandably, therefore, all the decisive technical advantages, unexpected by anyone skilled in the art, and listed hereinabove, are obtained according to the invention, and moreover, the proposed apparatus is very versatile and very easy to handle for a practitioner.

### BRIEF DESCRIPTION OF THE DRAWINGS

The invention will be more readily understood on reading the following description of several embodiments of an apparatus according to the invention for the surgical treatment of prostate tissues by thermal effect,
reference being made to the accompanying drawings, in which :
- Figure 1 is a diagrammatical view of a partial section of the complete apparatus according to the invention for the surgical treatment of tissues to be destroyed by thermal effect, illustrated in its preferred application to the treatment of the prostate, and comprising a urethral emitting probe means and a rectal echographic detecting probe means, both connected to a central control device ;
- Figure 2 is a view showing a partial longitudinal axial section of the main part of the urethral emitting probe means ;
- Figure 2a is a cross-section along line IIa-IIa of Figure 2 ;
- Figure 3 is a perspective side view of the urethral emitting probe means shown in Figures 1 and 2, the positioning means, preferably a urethral balloon being deflated ;
- Figure 4 illustrates a variant embodiment of the urethral emitting probe means according to the invention, showing a longitudinal axial section of a second embodiment of the cooling means ;
- Figure 5 illustrates another variant embodiment of the structural design of the urethral emitting probe means, showing an axial cross-section of another embodiment of the cooling means ;
- Figure 6 illustrates another variant of the cooling means in a view similar to Figure 5 ;
- Figure 7 illustrates yet another variant embodiment of the cooling means in a view similar to Figure 5.

### DETAILED DESCRIPTION OF PREFERRED EMBODIMENTS

Referring in particular to Figure 1, this shows an apparatus according to the invention, bearing the general reference number 10, for the surgical treatment of prostate tissues 12 by thermal effect, comprising heating means 16 for inducing thermal effect, consisting of a microwave generating device 18, clearly shown in Figure 2, placed in an emitting probe means of general reference number 20, shown in longitudinal axial section in Figure 2, adapted to be inserted into the urethra. The microwave generating device is capable of generating microwaves at a frequency of 1,300 MHz ± 50 MHz. Frequency setting means are also provided to set the frequency precisely at a frequency value within the range of 1,300 MHz ± 50 MHz.

According to a preferred embodiment, means for sensing the electrical field and/or means for sensing the temperature are present, preferably within a rectal probe means 82.

According to a specific preferred embodiment, the emitting probe means 20 comprises a front end 20a provided with positioning means 26 to position and maintain in position the urethral probe in the urethra. Preferably, said positioning means comprise a balloon means which can be of the Foley type which can be inflated or deflated at will with a liquid medium and which is helpful for inserting quickly the emitting probe, when it is a urethral one, when the balloon is deflated, into the urethra up to the level of the tissues to be treated by thermal effect, at wich level said balloon means reaches the bladder, so that, after inflating said balloon means with said liquid medium, the front end of the urethral probe 20 is locked in the bladder neck, thereby reaching a precise positioning of the microwaves antenna means with regard to the prostate and maintaining this positioning during the whole treatment as clearly seeen in Figure 1.

The apparatus according to a preferred embodiment of the invention is characterized in that it comprises heat protecting means 22, 30 for protecting the sensitive tissues other than the tissues 12 to be destroyed, such as the prostate 14, said means preferably comprising means forming radioreflecting screen 22, 30.

According to a variant embodiment of the invention, the means forming radioreflecting screen comprise a radioreflecting liquid medium, preferably an electrically conductive medium able to reflect the microwaves emitted by the emitting probe means 20 equipped with the microwave emitting device 18.

According to a particular variant of embodiment, said radioreflecting conductive solution is injected directly into the patient's bladder 24. In this case, said solution is preferably hypertonic. It can be, for example, a solution with a high ion concentration, such as by using NaCl. In general, suitable conductive solutions are saturated ionic solutions, such as NaCl or nitrate solutions, said solutions are obvious to anyone skilled in the art.

According to another variant of embodiment, one part 22 of the screen forming means is placed at the level of an organ to be protected, in particular the bladder. According to a preferred embodiment, said means forming protection screen for the bladder comprises said urethral balloon 26 provided on the front end 20a of the probe means 20. Said balloon 26 is equipped with inflating means 28 for inflating it with a liquid medium preferably with a radioreflecting conductive solution 23. Said inflating means normally comprise an internal duct 29 extending out towards a flexible tube 30. Said radioreflecting conductive solution may be identical to the previous one and based on saturated NaCl solution.

According to one advantageous embodiment of the emitting probe means according to the invention, said probe means comprises cooling means 32 incorporated to the probe means 20 for lowering the temperature of the external wall of said probe means 20, namely, the walls of the urethra 34. Said cooling means 32 consists, for example, in at least two conduits, respectivel an inlet conduit 36 and an outlet conduit 38, containing the cooling medium and communicating with each other at the front of the probe means 20 via a connection conduit 37 clearly shown in Figure 2. Said conduits 36, 38 communicate with flexible tubes 40, 42, respectively a supply tube and a discharge tube for the cooling medium, which may be water.

According to another characteristic of the invention, the urethral emitting probe means 20 is equipped with means 44 for sensing the temperature on or inside the urethral probe means 20. According to an advantageous variant, said temperature sensor 44 comprises a fiber-optic thermometer 46, the optical fibers being isolated by heat-insulating means 48 from the rest of the probe means and in particular from the cooling circuit 36, 38, as illustrated in Figures 2a and 3. Said thermometer comprises a plurality of strands of fibers placed in radially and/or longitudinally offset fashion in the longitudinal direction of the probe means 20, in such a way as to detect the temperature in various radial and/or longitudinal positions of the probe means.

Said thermometer 46 and associted insulating means 48 may be disposed in a longitudinal slot 50 provided in the external surface 21 of the probe means 20.

It will be noted that, advantageously, the front end 20a of the probe means 20 should be rounded to facilitate insertion of the probe means 20 through the urethra and up to the bladder 24, as can be seen in Figure 1.

The microwave generating device 16, as illustrated in Figures 2 and 3, comprises an inner conductor 52 constituted by a metallic cable coaxial to the probe means 20 and covered with an electrically insulating sleeve 53 over its whole length, and an outer conductor 54 extending over part of the length of the inner conductor. The emitting microwave antenna means is defined by the front part 52a of the inner conductor 52 free of outer conductor 54. The inner conductor has its front part 52a located at an intermediate part of the probe to leave space for the positioning means 22, the length of the front part 52a forming antenna means is preferably about one fourth of the microwave wavelength. Preferably, the free end of the cable 52a forming microwave emitting antenna ends up under the urethral balloon 26 forming the means protecting the bladder. Said cable 52 forms an emitting antenna which may be for example 20 to 40 mm long and which radiates in all directions about its axis, i.e. over 360°.

Said emitting antenna 52a constituted by the non-insulated part of the cable 52 is connected to a microwave generator apparatus symbolized by the initials M.W.G., emitting at 1300 MHz ± 50 MHz, and of power adjustable up to 50 W or even 100 W, thereby reaching a most effective thermal effect treatment of the tissues to be treated.

According to a variant of embodiment, the emitting antenna may be rather more complex and comprise shielding portions along the non-insulated part of cable 52, this with a view to preferentially heating certain parts of the prostate.

Figure 2a shows an axial cross-section of the emitting probe means 20, and illustrates the shape and position of each one of the elements constituting the probe means. This figure shows that the conduits 36, 38 containing the cooling medium, such as water, are formed by wide openings extending over an arc of circle of the probe means 20 which latter has a substantially circular or slightly ovoidal cross-section for easy passage through the urethra. It is observed that according to the variant embodiment illustrated in Figure 2a, the temperature sensor 44 and the duct 29 supplying the balloon 26 are situated in substantially the same plane passing through the axis of the probe means, hence of the cable 52, whereas the conduits 36, 38 containing the cooling medium are also arranged symmetrically with respect to said plane of symmetry.

Other variants of structure of the urethral probe means 20 are shown in Figures 4 to 7 in which the same reference numbers increased by 100, are used to designate identical parts or parts having the same function.

In the embodiment shown in Figure 4, the urethral probe means designated by the general reference 120 comprises an annular duct 136 coaxial to the probe means 120 communicating with a disc-shaped common duct 137 reaching to a central conduit 138 in which is introduced the microwave-emitting device 18.

Referring now to Figure 5, this shows another embodiment of the internal structure of the probe means, of which the general reference number has, in this example, been increased of 200, giving it the reference 220. As shown in this figure, the inlet conduit 236 supplying the cooling medium, occupies an annular sector of the probe means 220, whereas the outlet conduit 238 discharging the heated cooling medium, occupies a complementary annular sector, both annular sectors 236, 238 being separated by longitudinal transversal partitions 239, 241. The emitting device 218 is always placed axially with respect to the probe means 220. Figure 5 also shows the conduit 229 supplying the inflating medium, such as for example an electrically conductive liquid, to the balloon provided on the front end of the probe means 220, as well as the temperature sensor 244.

Figure 6 illustrates another variant of embodiment in which the parts which are identical or have the same function have been given the same reference numbers, further increased by 100. According to said Figure 6, four inlet conduits 336 supplying the cooling medium are provided, said conduits occupying annular sectors of the probe means 320 from the periphery 321 to the central longitudinal orifice 353 in which the microwave-emitting device 318 is inserted. The remaining sectors comprise corresponding annular conduits 338 for discharging the cooling medium, a conduit 329 being also provided for inflating the balloon as well as a slot 350 for housing the temperature sensor 344.

Referring to Figure 7, this shows yet another variant of the structure of the probe means of which the reference number has been further increased by 100, making it then 420. Four cooling medium inlet sectors 436 are also provided here, said sectors having rounded edges so that the remaining sectors 438 are substantially cross-shaped, which remaining cooling medium outlet sectors may be intercommunicating or not. The emitting device 418 is also placed in the axis of the probe means 428.

It is therefore understood that various forms of embodiment are possible for the structure of the emitting probe means. The probe means structure such as described and illustrated in Figures 1 through 7, forms a preferred embodiment.

The apparatus according to a preferred embodiment of the invention further comprises control means 80 for controlling the positioning of the urethral probe means 20, said control means 80 being preferably incorporated in a rectal probe means 82. Said control means 80 are preferably constituted by an echographic probe 82 of rectal type. Said echographic probe is advantageously connected with a screen-equipped echograph 84. In a variant embodiment, the echographic probe may be connected to a central control device 86 centralizing all the information and capable of automatically controlling the microwave generator device M.W.G. as a function of the recorded temperature data and of any data which may have been programmed by the practitioner, as can be understood from Figure 1.

The central control device 86, such as a computer or the like, receives all the temperature data centralized in a thermometry device as well as the temperature data from the cooling device supplying the cooling means of the emitting probe means 20 ; it also give the instructions necessary to the microwave generating device (M.W.G.) as well as to the cooling device with the purpose of either notifying the cooling flow and/or the cooling temperature. According to a variant embodiment, it can also receive the data issued from the echographic probe 82 relatively to the position of the emitting probe means 20 in relation to the prostate.

An intersticial thermometer 100 may also be provided, inserted in the prostate 14, under echographic control, so that when biopsies have to be carried out, it is possible to detect the temperature at which the prostate 14 has been brought by the microwaves emitted by the probe means 20. Such temperature data is of course important to determine the power instructions given by the control device 86 to the microwave generator M.W.G.

The tissues-protecting means according to the invention may comprise a part 90 fast with the rectal probe means 82, for protecting the rectal wall from high temperatures. According to a particularly advantageous embodiment, said screen-forming means 90 fast with the rectal wall from high temperatures. According to a particularly advantageous embodiment, said screen-forming means 90 fast with the rectal probe means comprise an rectal inflatable element 92 forming balloon fixed around the rectal probe 82 on its front end 82a which element is preferably supplied with an inflating liquid, advantageously a radioreflecting liquid medium 94, through a flexible tube 96 preferably identical to that used for inflating the urethral balloon 26 of the urethral probe means. Several temperature sensors 98 may be provided on the rectal probe or inside the balloon 92 for detecting the temperature at the level of the rectal wall 100 facing the prostate 14, the temperature data recorded by said sensors being transmitted to the thermometry device and to `he central control device 86, such as a computer or the like.

In particular, said rectal temperature sensors can be used to measure the temperature reaching the rectal wall and to sense the probe of temperature and/or of microwave field generated by the microwaves as a means for detecting a correct position of the rectal probe and/or of the urethral probe.

According to a variant embodiment of the invention, said screen-forming means may comprise at least one wire, parallel to the mean electrical field, which wire is placed preferably in integral fashion with the rectal probe means 82 longitudinally thereto, in the direction of the emitting probe means 20.

Means may also be provided for protecting the sphincter 15 by a balloon system surrounding the probe means 20 similarly to balloon 26, which system can also be supplied by supply means 30.

It is obviously possible, according to the invention, to treat efficiently the prostate 14 by thermal effect, and in general to destroy tissues by thermal effect.

It is extremely easy with the echographic probe 82 connected with a screen-equipped echograph 84 to control the exact position of the emitting probe means 20 inside the prostate 14 by monitoring the echographic image of the emitting probe means 20 on the screen 84. When the practitioner sees that the emitting probe means is accurately positioned in the prostate 14, he can then trigger manually the control device 86 into operation, for carrying out the thermal effect treatment, the temperature being controlled inside the prostate 14 as well as in the surrounding tissues, and in particular in the rectum, by the various temperature sensors such as 98, 100.

It is also possible with the apparatus according to the invention, to protect efficiently the tissues other than the prostate, by various protection means 22, 90. Furthermore, the presence of the cooling means 32 prevents burn form occuring on the walls in contact with the probe means 20, particularly the walls of the urethra 34.

The apparatus according to the invention is useable in a method for surgically treating prostate tissues 12 by thermal effect, using a microwave generating device 18 provided in an emitting probe means 20 capable of being introduced into the urethra, wherein the sensitive tissues other than the tissues 12 to be treated are protected from the heat preferably by providing means forming radioreflecting screen 22, 90.

According to a variant embodiment of this surgical treatment method, said urethral emitting probe means 20 is inserted in the urethra very quickly up to a level for which the urethral balloon 26 which is of course deflated, reaches the bladder, then the urethral balloon 26 is inflated and a withdrawal movement of the urethral probe 20 is performed to lock the urethral probe 20 in the bladder neck.

It is preferably provided that one part 22 of said screen-forming means is placed at the level of an organ to be protected, such as the bladder 24 and/or the sphincter 15. This protection can be easily provided by filling the urethral ballon 26 with a cooling liquid and in particular an electroconductive liquid which provides radioreflection.

According to another advantageous variant of the method of the invention, the positioning of the urethral probe means 20 in relation to the prostate 14 is controlled by inserting a rectal probe means 82 in the rectum.

Advantageously, a protection forming radioreflecting means is also provided for protecting the rectal wall 100 from high temperatures.

This protection is ensured by the rectal balloon 92 inflated with a cooling liquid, in particular an electroconductive liquid.

The power of the microwave and/or the flow of the cooling liquid can be adjusted so as to maintain the urethral temperature below 44.5°C and the rectal temperature below 42.5°C.

By way of indication, the total duration of a treatment of the prostate by hyperthermia is normally about 1 hour. The emitting probe means will preferably emit microwaves at a frequency of about 1300 MHz ± 50 MHz and at a power which can be adjusted up to 50 W or even 100 W. The length of the actual emitting antenna is for example between 20 and 40 mm, these values being given by way of indication only. To work at the old frequency of 915 MHz is less efficient than with the invention frequency of 1300 MHz ± 50 MHz.

According to a specific embodiment, dilation means for temporary or intermittently increasing the outside diametre of the urethral probe are provided, said means being disposed relatively to the probe in such a manner as to enable the dilation of the urethral walls, at least at the level where the tissues are treated, and when so desired. In particular, said dilation means comprise at least one dilation balloon having means for filling and/or emptying the dilation balloon.

## Claims

1. An apparatus (10) for the surgical treatment of prostate tissues by thermal effect, comprising an urethral emitting probe (16) adapted to be inserted in the urethra, having a front end and a rear end, said emitting probe comprising a microwave antenna means (18) located intermediate or in the vicinity of said front end, said microwave antenna means being connected to an external microwave generating device (M.W.G.), said urethral emitting probe comprising cooling means (32, 36, 38, 40, 42) fed with a cooling medium, and being capable of preventing burns on the walls of the prostatic urethra in contact with the urethral probe means, said external microwave generating device comprises means for generating microwaves at a frequency of 1300 MHz ± 50 MHz, and a power effective to heat permanently said tissues to a predetermined temperature for a period of time sufficient to treat said tissues by thermal effect.

2. The apparatus of claim 1, wherein said cooling means (32, 36, 38, 40, 42) are extending up to the full length of the microwave antenna means.

3. The apparatus of claim 1 or 2, wherein an inflatable balloon means (26) is located between said microwave antenna means (18) and said front end (20a), and means for inflating said balloon means (28), said balloon means reaching the bladder when said urethral probe is positioned in the urethra.

4. The apparatus of claim 2, wherein said means for inflating said balloon means comprise an electroconductive liquid forming a radio reflecting screen capable of reflecting the microwaves emitted by said microwave antenna means, thereby protecting the bladder from being damaged by said microwaves.

5. The apparatus of anyone of claims 1 to 3, wherein said power is effective to treat said tissues by heat energy at a temperature higher than 45°C and preferably ranging about 45°C and not more than 85°C, during a period of time sufficient to reach a necrosis of the tissues submitted to the thermal effect, to perform thermotherapy.

6. The apparatus of anyone of claims 1 to 3, wherein said power is effective to treat said tissues by heat energy at a temperature range lower than 45°C and generally of 40°C-43°C to perform a hyperthermia treatment.

7. The apparatus of anyone of claims 1 to 6 comprising:
a) a urethral emitting probe (20) having a front end and a rear end, capable of being inserted in the urethra, said urethral emitting probe comprising a microwave antenna means located intermediate or in the vicinity of said front end, said microwave antenna means being connected to an external microwave generating device (M.W.G.);
b) a rectal probe means (82) comprising control means (80) for controlling the positioning of the urethral probe means;
c) a central control device (86) receiving the information provided by the control means for controlling the positioning of the urethral probe means,
d) correcting means for correcting the positioning of the urethral probe means, and
e) said external microwave generating device being capable of generating microwaves at said frequency and at a power effective to heat said tissues to a predetermined temperature, for a period of time sufficient to treat said tissues by thermal effect.

8. The apparatus of claim 7, wherein said rectal probe comprises an echographic probe (82) connected to a image forming device (84) to facilitate the positioning of the urethral probe means, thereby having an echographic control of the surgical treatment of said tissues.

9. The apparatus of claims 7 or 8, wherein said urethral probe comprises at least one urethral temperature sensor (100) located in the vicinity of the microwave antenna means for detecting urethral temperature,
- said rectal probe comprises at least one rectal temperature sensor (98) for detecting the rectal temperature at the level of the rectal wall facing the tissues to be treated, and transmission means to transmit said temperature data recorded by said urethral and rectal temperature sensors to a thermometry device and to said central control device (86),
- said central control device comprises power and cooling temperature adjusting means to adjust one or both of the power of the microwaves and of the cooling temperature of the cooling means, when the temperature detected is not conform to a pretermined temperature range.

10. The apparatus of claim 9, wherein said rectal probe comprises liquid medium feeding means (96) for feeding a liquid medium, capable of providing heat protection of the rectal walls.

11. The apparatus of claim 9 or 10, wherein said rectal probe is provided with radio reflecting means (94) to protect the rectal walls from heat.

12. The apparatus of anyone of claims 9 to 11, wherein said urethral temperature sensor comprises a plurality of strands of optical fibers placed in radially and/or longitudinally offset fashion in the longitudinal direction of the urethral emitting probe, preferably said urethral temperature sensor being located within a longitudinal slot provided in the external surface of the probe means.

13. The apparatus of anyone of the preceding claims wherein the urethral probe means comprises at least two cooling conducts (36, 38), communicating with each other at the front end (20a) of the probe means via a connection conduct and at a rear end respectively with a supply tube (40) and a discharge tube (42) with the cooling medium.

14. The apparatus of anyone of claims 1 to 13, wherein the microwave antenna means comprises an inner conductor (52) comprising a metallic cable coaxial to the urethral probe covered by an electrically insulating sleeve over its whole length, and an outer conductor (54) extending over part of the length of the inner conductor, the emitting microwave antenna means being defined by the front part (52a) of the inner conductor, which ends to an intermediate level of the emitting probe means.

15. The apparatus of claim 14, wherein the front part of the inner conductor ends up under the balloon means (26).

16. The apparatus of anyone of claims 9 to 15, comprising safety devices including a thermometry feed-back system which maintains the temperature at not more than 44.5°C at the level of the urethra walls as measured by the urethral thermosensors, and at not more than 42.5°C, at the level of the rectal walls as measured by the rectal thermosensor, said power being adjustable up to 100 watts.

17. The apparatus of claims 1 to 16, wherein the urethral probe comprises dilation means for temporary or intermittently increasing the outside diametre of the urethral probe, said means being disposed relatively to the probe in such a manner as to enable the dilation of the urethral walls, at least at the level where the tissues are treated, and when so desired ; in particular, said dilation means comprise at least one dilation balloon having means for filling and/or emptying the dilation balloon.

## Patentansprüche

1. Vorrichtung (10) zur chirurgischen Behandlung von Prostatageweben durch Wärmewirkung, mit einer Harnröhren-Emissionssonde (16) zum Einführen in die Harnröhre, welche ein vorderes und ein hinteres Ende hat, wobei die Emissionssonde ein Mikrowellenantennenmittel (18) dazwischen oder in der Nähe des vorderen Endes aufweist, wobei das Mikrowellenantennenmittel mit einer äußeren Mikrowellen-Generatoreinrichtung (M.W.G.) verbunden ist, wobei die Harnröhren-Emissionssonde mit einem Kühlmedium gespeiste Kühlmittel (32, 36, 38, 40, 42) aufweist und Verbrennungen an den Wänden der prostatischen Harnröhre, die mit dem Harnröhrensondenmittel in Kontakt sind, verhindern kann, wobei die äußere Mikrowellen-Generatoreinrichtung Mittel zur Erzeugung von Mikrowellen mit einer Frequenz von 1300 MHz ± 50 MHz und eine Leistung hat, die wirksam ist, um die Gewebe dauerhaft auf eine vorbestimmte Temperatur für eine Zeitdauer zu erhitzen, die ausreicht, um die Gewebe durch Wärmewirkung zu behandeln.

2. Vorrichtung nach Anspruch 1, worin die Kühlmittel (32, 36, 38, 40, 42) sich bis zur gesamten Länge des Mikrowellenantennenmittels erstrecken.

3. Vorrichtung nach Anspruch 1 oder 2, worin ein aufblasbares Ballonmittel (26) zwischen dem Mikrowellenantennenmittel (18) und dem vorderen Ende (20a) angeordnet ist und mit Mitteln zum Aufblasen des Ballons (28), wobei der Ballon die Blase erreicht, wenn die Harnröhrensonde in der Harnröhre positioniert ist.

4. Vorrichtung nach Anspruch 2, worin die Mittel zum Aufblasen des Ballons eine elektrisch leitende Flüssigkeit aufweisen, welche einen strahlenreflektierenden Schirm bildet, der die vom Mikrowellenantennenmittel emittierten Mikrowellen reflektieren kann, wodurch die Blase vor einer Beschädigung durch die Mikrowellen geschützt ist.

5. Vorrichtung nach einem der Ansprüche 1 bis 3, worin die Leistung wirksam ist, um die Gewebe durch Wärmeenergie mit einer Temperatur von mehr als 45°C und vorzugsweise im Bereich von etwa 45°C und nicht mehr als 85°C während einer Zeitdauer zu behandeln, die ausreicht, um eine Nekrose der der Wärmewirkung ausgesetzten Gewebe zu erreichen, um eine Thermotherapie durchzuführen.

6. Vorrichtung nach einem der Ansprüche 1 bis 3, worin die Leistung wirksam ist, um die Gewebe durch Wärmeenergie in einem Temperaturbereich von weniger als 45°C und im allgemeinen von 40°C-43°C zu behandeln, um eine Hyperthermiebehandlung durchzuführen.

7. Vorrichtung nach einem der Ansprüche 1 bis 6, welche aufweist:
a) eine Harnröhren-Emissionssonde (20) mit einem vorderen Ende und einem hinteren Ende, die in die Harnröhre eingeführt werden kann, wobei die Harnröhren-Emissionssonde ein Mikrowellenantennenmittel dazwischen oder in der Nähe des vorderen Endes aufweist, wobei das Mikrowellenantennenmittel mit einer äußeren Mikrowellen-Generatoreinrichtung (M.W.G.) verbunden ist,
b) ein rektales Sondenmittel (82) mit Steuermitteln (80) zur Steuerung der Positionierung der Harnröhren-Sondenmittel;
c) eine zentrale Steuereinrichtung (86), welche die vom Steuermittel zur Steuerung der Positionierung der Harnröhren-Sondenmittel gelieferte Information empfängt,
d) Korrekturmittel zum Korrigieren der Positionierung der Harnröhren-Sondenmittel, und
e) wobei die äußere Mikrowellen-Generatoreinrichtung Mikrowellen mit dieser Frequenz und mit einer Leistung erzeugen kann, die wirksam ist, um die Gewebe auf eine vorbestimmte Temperatur während einer Zeitdauer zu erwärmen, die ausreicht, um die Gewebe durch Wärmewirkung zu behandeln.

8. Vorrichtung nach Anspruch 7, worin die rektale Sonde eine Echographsonde (82) aufweist, die mit einer Bilderzeugungseinrichtung (84) verbunden ist, um die Positionierung der Harnröhrensondenmittel zu erleichtern, wodurch eine echographische Steuerung der chirurgischen Behandlung der Gewebe gegeben ist.

9. Vorrichtung nach den Ansprüchen 7 oder 8, worin die Harnröhrensonde mindestens einen Harnröhrentemperaturmeßfühler (100) aufweist, welcher sich in der Nähe des Mikrowellenantennenmittels befindet, um die Harnröhrentemperatur zu erfassen,
- wobei die rektale Sonde mindestens einen Rektaltemperaturmeßfühler (98) zum Erfassen der Rektaltemperatur in der Höhe der dem zu behandelnden Gewebe zugewandten Rektalwand und Übertragungsmittel zum Übertragen der von den Harnröhren- und Rektaltemperaturmeßfühlern aufgezeichneten Temperaturdaten zu einer Temperaturmeßeinrichtung und zur zentralen Steuereinrichtung (86) aufweist,
- wobei die zentrale Steuereinrichtung Leistungs- und Kühltemperatureinstellmittel zur Einstellung von einem oder von beiden von Leistung der Mikrowellen und Kühltemperatur der Kühlmittel aufweist, wenn die detektierte Temperatur nicht mit einem vorbestimmten Temperaturbereich übereinstimmt.

10. Vorrichtung nach Anspruch 9, worin die Rektalsonde Flüssigkeitsmediumzuführmittel (96) zum Zuführen eines flüssigen Mediums, das einen Wärmeschutz für die Rektalwände bieten kann, aufweist.

11. Vorrichtung nach Anspruch 9 oder 10, worin die Rektalsonde mit strahlenreflektierenden Mitteln (94) versehen ist, um die Rektalwände vor Wärme zu schützen.

12. Vorrichtung nach einem der Ansprüche 9 bis 11, worin der Harnröhrentemperaturmeßfühler eine Mehrzahl von Lichtleiterbündeln aufweist, die radial und/oder in Längsrichtung versetzt, in der Längsrichtung der Harnröhren-Emissionssonde angeordnet sind, wobei sich der Harnröhrentemperaturmeßfühler vorzugsweise in einem in der Außenfläche der Sondenmittel vorgesehenen Längsschlitz befindet.

13. Vorrichtung nach einem der vorhergehenden Ansprüche, worin das Harnröhrensondenmittel mindestens zwei Kühlleitungen (36, 38) aufweist, die am vorderen Ende (20 a) des Sondenmittels miteinander über eine Verbindungsleitung bzw. an einem hinteren Ende mit einem Zuführschlauch (40) und einem Ableitungsschlauch (42) mit dem Kühlmedium kommunizieren.

14. Vorrichtung nach einem der Ansprüche 1 bis 13, worin das Mikrowellenantennenmittel einen inneren Leiter (52), der ein Metallkabel hat, welches zur Harnröhrensonde koaxial verläuft und über seine gesamte Länge von einer elektrisch isolierenden Hülse überzogen ist, und einen äußeren Leiter (54), der sich über einen Teil der Länge des inneren Leiters erstreckt, aufweist, wobei das Emissionsmikrowellenantennenmittel durch den vorderen Teil (52a) des inneren Leiters definiert ist, welcher innere Leiter auf mittlerer Höhe des Emissionssondenmittels endet.

15. Vorrichtung nach Anspruch 14, woirn der vordere Teil des inneren Leiters unter dem Ballonmittel (26) endet.

16. Vorrichtung nach einem der Ansprüche 9 bis 15 mit Sicherheitseinrichtungen, einschließlich eines Temperaturmessungs-Feedback-Systems, welches die von den Harnröhren-Wärmemeßfühlern gemessene Temperatur auf nicht mehr als 44,5°C auf der Höhe der Harnröhrenwände hält, wobei die Leistung auf bis zu 100 Watt einstellbar ist.

17. Vorrichtung nach den Ansprüchen 1 bis 16, worin die Harnröhrensonde Dilatationsmittel zum vorübergehenden oder intermittierenden Vergrößern des Außendurchmessers der Harnröhrensonde aufweist, wobei diese Mittel in bezug auf die Sonde so angeordnet sind, daß sie die Dilatation der Harnröhrenwände mindestens auf der Höhe, auf welcher die Gewebe behandelt werden, auf Wunsch ermöglichen; wobei die Dilatationsmittel insbesondere mindestens einen Dilatationsballon mit Mitteln zum Füllen und/oder Entleeren des Dilatationsballons umfassen.

## Revendications

1. Appareil (10) de traitement chirurgical de tissus prostatiques par effet thermique, comprenant une sonde urétrale émettrice (16) conçue pour être insérée dans l'urètre, ayant une extrémité avant et une extrémité arrière, ladite sonde émettrice comprenant un moyen formant antenne de micro-ondes (18) situé dans une position intermédiaire ou à proximité de ladite extrémité avant, ledit moyen formant antenne de micro-ondes étant connecté à un dispositif externe de génération de micro-ondes (G.M.O.), ladite sonde urétrale émettrice comprenant des moyens de refroidissement (32, 36, 38, 40, 42) alimentés en un milieu refroidissant, et étant capable de prévenir des brûlures sur les parois de l'urètre prostatique en contact avec le moyen formant sonde urétrale, ledit dispositif externe de génération de micro-ondes comprenant un moyen destiné à générer des micro-ondes à une fréquence de 1300 MHz ± 50 MHz et à une puissance efficace pour chauffer de façon permanente lesdits tissus à une température prédéterminée pendant une durée suffisante pour traiter lesdits tissus par effet thermique.

2. Appareil selon la revendication 1, dans lequel lesdits moyens de refroidissement (32, 36, 38, 40, 42) s'étendent sur toute la longueur du moyen formant antenne de micro-ondes.

3. Appareil selon la revendication 1 ou 2, dans lequel un moyen formant ballonnet gonflable (26) est situé entre ledit moyen formant antenne de micro-ondes (18) et ladite extrémité avant (20a), et un moyen (28) destiné au gonflement dudit moyen formant ballonnet, ledit moyen formant ballonnet atteignant la vessie lorsque ladite sonde urétrale est positionnée dans l'urètre.

4. Appareil selon la revendication 2, dans lequel ledit moyen destiné au gonflement dudit moyen formant ballonnet comprend un liquide électriquement conducteur formant un écran radioréfléchissant capable de réfléchir les micro-ondes émises par ledit moyen formant antenne de micro-ondes, protégeant ainsi la vessie de l'endommagement par lesdites micro-ondes.

5. Appareil selon l'une quelconque des revendications 1 à 3, dans lequel ladite puissance est efficace pour traiter lesdits tissus par énergie thermique à une température supérieure à 45°C et de préférence comprise entre environ 45°C et pas plus de 85°C, pendant une durée suffisante pour atteindre la nécrose des tissus soumis à l'effet thermique, pour réaliser une thermothérapie.

6. Appareil selon l'une quelconque des revendications 1 à 3, dans lequel ladite puissance est efficace pour traiter lesdits tissus par énergie thermique à une gamme de températures inférieure à 45°C et généralement de 40°C à 43°C, pour réaliser un traitement par hyperthermie.

7. Appareil selon l'une quelconque des revendications 1 à 6, comprenant :
a) une sonde urétrale émettrice (20) ayant une extrémité avant et une extrémité arrière, capable d'être insérée dans l'urètre, ladite sonde urétrale émettrice comprenant un moyen formant antenne de micro-ondes situé dans une position intermédiaire ou à proximité de ladite extrémité avant, ledit moyen formant antenne de micro-ondes étant connecté à un dispositif externe de génération de micro-ondes (G.M.O.);
b) un moyen formant sonde rectale (82) comprenant un moyen de contrôle (80) destiné à contrôler le positionnement du moyen formant sonde urétrale;
c) un dispositif de contrôle central (86) recevant l'information fournie par le moyen de contrôle destiné à contrôler le positionnement du moyen formant sonde urétrale ;
d) des moyens de correction destinés à corriger le positionnement du moyen formant sonde urétrale et
e) ledit dispositif externe de génération de micro-ondes étant capable de générer des micro-ondes à ladite fréquence et à une puissance efficace pour chauffer lesdits tissus à une température prédéterminée, pendant une durée suffisante pour traiter lesdits tissus par effet thermique.

8. Appareil selon la revendication 7, dans lequel ladite sonde rectale comprend une sonde échographique (82) connectée à un dispositif de formation d'image (84) pour faciliter le positionnement du moyen formant sonde urétrale, ayant ainsi un contrôle échographique du traitement chirurgical desdits tissus.

9. Appareil selon les revendications 7 à 8, dans lequel ladite sonde urétrale comprend au moins un détecteur de température urétrale (100) situé à proximité du moyen formant antenne de micro-ondes pour détecter la température urétrale,
- ladite sonde rectale comprend au moins un détecteur de température rectale (98) destiné à détecter la température rectale au niveau de la paroi rectale orientée vers les tissus à traiter, et un moyen de transmission pour transmettre lesdites données de température enregistrées par lesdits détecteurs de température urétrale et de température rectale à un dispositif de thermométrie et audit dispositif de contrôle central (86),
- ledit dispositif de contrôle central comprend des moyens d'ajustement de la puissance et de la température de refroidissement pour ajuster la puissance des micro-ondes et/ou la température de refroidissement du moyen de refroidissement, lorsque la température détectée n'est pas conforme à une gamme de températures prédéterminée.

10. Appareil selon la revendication 9, dans lequel ladite sonde rectale comprend un moyen d'apport de milieu liquide (96) destiné à apporter un milieu liquide, capable d'assurer la protection thermique des parois rectales.

11. Appareil selon la revendication 9 ou 10, dans lequel ladite sonde rectale est dotée de moyens radioréfléchissants (94) pour protéger les parois rectales de la chaleur.

12. Appareil selon l'une quelconque des revendications 9 à 11, dans lequel ledit détecteur de température urétrale comprend une pluralité de brins de fibres optiques placés en décalage radial et/ou longitudinal dans la direction longitudinale de la sonde urétrale émettrice, ledit détecteur de température urétrale étant de préférence situé à l'intérieur d'une fente longitudinale ménagée dans la surface externe du moyen formant sonde.

13. Appareil selon l'une quelconque des revendications précédentes, dans lequel le moyen formant sonde urétrale comprend au moins deux conduits de refroidissement (36, 38) communiquant entre eux à l'extrémité avant (20a) du moyen formant sonde par un conduit de connexion et à une extrémité arrière avec respectivement un tube d'apport (40) et un tube d'évacuation (42) de milieu refroidissant.

14. Appareil selon l'une quelconque des revendications 1 à 13, dans lequel le moyen formant antenne de micro-ondes comprend un conducteur interne (52) comprenant un câble métallique coaxial à la sonde urétrale, couvert par une gaine électriquement isolante sur toute sa longueur et un conducteur externe (54) s'étendant sur une partie de la longueur du conducteur interne, le moyen formant antenne émettrice de micro-ondes étant défini par la partie avant (52a) du conducteur interne, qui se termine à un niveau intermédiaire du moyen formant sonde émettrice.

15. Appareil selon la revendication 14, dans lequel la partie avant du conducteur interne se termine sous le moyen formant ballonnet (26).

16. Appareil selon l'une quelconque des revendications 9 à 15, comprenant des dispositifs de sécurité incluant un système d'asservissement par thermométrie qui maintient la température à un chiffre inférieur ou égal à 44,5°C au niveau des parois de l'urètre, tel que mesuré par les thermodétecteurs urétraux, et à un chiffre inférieur ou égal à 42,5°C au niveau des parois rectales, tel que mesuré par le thermodétecteur rectal, ladite puissance étant ajustable jusqu'à 100 watts.

17. Appareil selon les revendications 1 à 16, dans lequel la sonde urétrale comprend un moyen de dilatation destiné à augmenter de façon temporaire ou intermittente le diamètre externe de la sonde urétrale, ledit moyen étant disposé par rapport à la sonde de façon à permettre la dilatation des parois urétrales, au moins au niveau où les tissus sont traités, et quand cela est désiré ; en particulier, ledit moyen de dilatation comprend au moins un ballonnet de dilatation comportant des moyens destinés à emplir et/ou vider le ballonnet de dilatation.
